# EUROPEAN PATENT SPECIFICATION

(11) **EP 3 302 711 B1**
(45) Date of publication and mention of the grant of the patent: **08.05.2019**
(21) Application number: 16739256.2
(22) Date of filing: 03.06.2016
(51) Int. Cl.: A61Q 1/12, A61Q 19/10, A61K 8/895, A61K 8/02

(54) **POWDER COSMETIC COMPOSITION, USES AND PROCESS FOR THE PREPARATION THEREOF**
PULVERFÖRMIGE KOSMETISCHE ZUSAMMENSETZUNG, VERWENDUNG UND DEREN HERSTELLUNG
COMPOSITION COSMÉTIQUE PULVÉRULANTE, UTILISATION ET PROCEDÉ DE PRÉPARATION

(30) Priority: 05.06.2015 IT UB20151027
(43) Date of publication of application: 11.04.2018
(73) Proprietor: B. Kolormakeup & Skincare S.p.A., 24047 Treviglio (BG) (IT)
(72) Inventor: DE LUIGI, Mario, 20129 Milano (IT)
(74) Representative: Marturano, Pasqualino
(86) International application number: PCT/IB2016/053266
(87) International publication number: WO 2016/193938

(56) References cited:
- DE-A1- 19 745 964
- GB-A- 2 423 250
- "Dow Corning 9701 Cosmetic Powder product information", , 29 September 2010 (2010-09-29), XP055019050, Retrieved from the Internet: URL:http://www2.dowcorning.com/DataFiles/0 90007c8802654fe.pdf [retrieved on 2012-02-10]
- DATABASE GNPD [Online] MINTEL; May 2013 (2013-05), XP002754772, Database accession no. 2067832
- DATABASE GNPD [Online] MINTEL; October 2011 (2011-10), XP002754773, Database accession no. 1646250

## Description

The present invention relates to a powder cosmetic composition, in particular for the cleansing and/or caring of the skin and hair, and uses and process for the preparation thereof.

In a particularly advantageous embodiment of the present invention, the cosmetic composition is in compact powder form, for example, in the form of a tablet.

The cosmetic composition of the present invention can be applied to both the skin and hair. Before application, the composition is hydrated to form a foam having a consistency of a soft cream to the touch.

As is known, compositions for skin cleansing are generally formulated in the form of solid bars, for example soaps, or in the form of more or less viscous liquids.

After repeated use, soaps lose their original geometrical form conferred in the production phase and become reduced in size. Used soaps frequently break up into small pieces, which the user has difficulty in adopting. A further disadvantage of solid soaps lies in their difficulty of use encountered by the users, especially children, when they have to handle a wet and therefore slippery soap.

In particular contexts, for example in hotel rooms, the use of solid soaps also implies the production of high quantities of unused residues. Even if smallsized, the solid soaps, in fact, are generally only partly used and must be replaced whenever a room is prepared for a subsequent user.

The disadvantages connected to the use of solid soaps have been partly overcome by liquid soaps. With respect to liquid soaps, however, the more fluid they are, the more difficult they are to dose, as they have the tendency to slip between the fingers. Liquid soaps also tend to leak from the container in which they are packaged, for example dripping from the dosing device, also after use. These losses can cause fouling of the surface on which the container is resting or of the user's clothing.

Compositions for cleansing and skin care in the form of a deformable solid are also known in the state of the art. US 6245343 describes a cosmetic composition comprising a binder in liquid form and a powder filler. The powder filler is present in a quantity which is sufficient for structuring the composition, giving it the consistency of a deformable solid, i.e. having a consistency which is such as to allow it to be modelled with the hands, for example like plasticine. The powder filler includes solid particles of a thermoplastic polymer and at least one powder surfactant. Before application, the composition is hydrated so as to form a foam which can be spread on the skin.

The main aim of the present invention is to overcome the drawbacks revealed in the state of the art associated with cosmetic compositions for the cleansing and/or treatment of the skin in solid and liquid form.

Within this general aim, a first objective of the present invention is to provide a powder cosmetic composition, particularly suitable for the cleansing and/or caring of the skin and hair, which is applied in the form of a foam having the consistency of a creamy fluid to the touch that can be easily rinsed.

A second objective of the present invention is to provide a compact powder cosmetic composition that is packaged in a single dose (monodose), so as to avoid, or at least reduce, the formation of waste, as it typically occurs with solid soaps.

The Applicant has now found that the above and other objectives that will appear more evident in the present description hereunder, can be achieved, according to a first aspect of the present invention, by means of a powder cosmetic composition that comprises the following components (weight percentages referring to the weight of the cosmetic composition):
(a) 60-95% by weight of an effervescent system comprising at least one carbonate or bicarbonate salt of an alkaline metal or alkaline earth metal capable of reacting with at least one acid component, when exposed to water, developing a gas;
(b) 1-35% by weight of at least one solid surfactant;
(c) 0.05-5% by weight, preferably within the range of 0.1-1%, more preferably within the range of 0.15-0.5%, of at least one composite powder consisting of particles of a crosslinked organosiloxane polymer supporting silica particles on the surface.

According to a second aspect, the present invention relates to a method for cleansing and/or caring the skin and/or hair, which comprises the following steps:
- hydrating the above said cosmetic composition so as to form a foam;
- applying said foam to the skin and/or hair;
- rinsing the skin and/or hair.

According to a third aspect, the present invention relates to a process for preparing the above cosmetic composition which comprises a step (i) of mixing the following components (percentages by weight referring to the weight of the cosmetic composition):
(a) 60-95% by weight of at least one powder effervescent system comprising at least one carbonate or bicarbonate salt of an alkaline metal or alkaline earth metal capable of reacting with at least one acid component, when exposed to water, developing a gas,
(b) 1-35% by weight of at least one solid surfactant,
(c) 0.05-5% by weight, preferably within the range of 0.1-1%, more preferably within the range of 0.15-0.5%, of at least one composite powder consisting of particles of a crosslinked organosiloxane polymer supporting silica particles on the surface,
to form said powder cosmetic composition.

According to a preferred embodiment, the above process also comprises a step (ii) for compacting the above powder cosmetic composition to obtain a cosmetic composition in a self-supporting compact form, preferably in the form of a tablet.

For the purposes of the present description and relative claims, the verb "comprise" and all the terms deriving therefrom also include the meaning of the verb "consist" and the terms deriving therefrom.

Further characteristics and advantages of the present invention will appear more evident from the following description.

The cosmetic composition according to the present invention (hereafter also simply indicated as "composition") is preferably destined to be used for the cleansing and/or cosmetic care of the human skin or hair. Skin that can be treated with the cosmetic composition can be the skin of the face and also of the body. Products destined for these uses are generally indicated on the market as "skin care" products.

The composition can also be advantageously destined for other uses, for example for the cleansing and/or cosmetic care of the hair.

The composition according to the present invention is in powder form, and can therefore be easily dosed by a user.

In a particularly preferred embodiment, the composition is in compact powder form (for example, a tablet or bar). When it is in compact powder form, the composition is self-supporting, i.e. it supports its own weight without becoming deformed and without the help of supporting or containment structures, such as, for example casings, capsules or bottoms.

The composition in self-supporting form offers the advantage of being easily manageable by a user, in particular in the phase prior to the application of the foam.

The composition according to the present invention, in particular in the self-supporting form, can be advantageously prepared and packaged in monodose portions which are not only safer from a hygienic point of view, but can also be entirely used in a single application on the skin or hair, thus avoiding the production of unused residues, contrary to what happens with the solid soaps of the known art.

The composition can also be prepared and packaged for use as a fluid powder, i.e. in a non-compact and non-self-supporting form, for example in sachets or bags, also in this case available in monodose portions.

According to the present invention, the powder composition is substantially anhydrous.

For the purposes of the present invention, the term "substantially anhydrous composition" means that the water content of the composition is lower than 5% by weight with respect to the weight of the composition, preferably lower than 3% by weight.

Before application on the skin or hair, the composition is hydrated by adding a quantity of water which is sufficient for forming a foam, which has the consistency of a creamy fluid and is particularly smooth-flowing on the skin. The foam can be easily removed by rinsing the skin with water. The application of the foam on the skin leaves a pleasant sensation of hydration, cleanliness and freshness on the user.

Thanks to the presence of one or more surfactants, the composition exerts a cleansing effect on the skin or hair. The composition can also be used as a means for conveying additional active ingredients on the skin or hair (for example, fragrances, emollients, conditioning agents, dermatological active ingredients, etc.), as these can be optionally added to the composition.

The cosmetic composition according to the present invention comprises an effervescent system in powder form, substantially anhydrous (the water content in the effervescent system is preferably lower than 5% by weight, more preferably lower than 3% by weight).

The effervescent system comprises at least one carbonate or bicarbonate salt of at least one alkaline metal or alkaline earth metal (base component) mixed with at least one acid component.

Examples of carbonates and bicarbonates that can be used as base component are: sodium carbonate, potassium carbonate, magnesium carbonate, calcium carbonate, sodium bicarbonate, potassium bicarbonate, magnesium bicarbonate, calcium bicarbonate and mixtures thereof.

The acid component can be selected from various cosmetically acceptable organic or inorganic acids, such as, for example: citric acid, tartaric acid, ascorbic acid, malic acid, maleic acid, glutamic acid, glycolic acid, salicylic acid and mixtures thereof.

When the above salts and acid component come into contact with water, they react forming carbonic acid and salts. Carbonic acid is unstable in water and releases CO₂ in gaseous form, thus creating the effervescent effect.

The stoichiometric ratio between the base component and the acid component preferably ranges from 3:1 to 1:3 (base equivalents : acid equivalents).

The effervescent system is preferably present in the cosmetic composition in a quantity within the range of 60-95% by weight with respect to the weight of components (a), (b) and (c), preferably within the range of 70-90% by weight. The combination of components (a), (b) and (c) is also indicated hereunder as "base composition".

The cosmetic composition also comprises at least one surfactant in powder form. The surfactant can be an anionic, non-ionic, cationic or amphoteric surfactant.

The overall quantity of surfactants present in the composition is preferably within the range of 1-35% by weight referring to the weight of the base composition, preferably within the range of 5-20% by weight.

Examples of anionic surfactants that can be used in the present invention are: polyalkylene glycol ethers of fatty alcohols, taurates, acyl lactylates, sodium stearoyl lactylate, alkyl sulfates, sodium lauryl sulfate, polyoxyethylenated alkyl sulfates, alkyl ether sulfate, monoethanolamine lauryl ether sulfate, alkyl ether carboxylate, monoalkyl phosphate, dialkyl phosphate, arginine mono-(2-hexyldecyl) phosphate, ethoxylated alkyl phosphate, N-acyl sarcosinate, sodium lauroyl sarcosinate, sodium myristoyl sarcosinate, N-acylglutamate, sodium lauroyl glutamate, acetyl isethionate, sodium cocoyl isethionate, polysorbate, potassium laurate, potassium myristate, potassium palmitate, potassium stearate and mixture thereof.

In a preferred embodiment, the composition comprises sodium lauryl sulfosuccinate and sodium cocoyl isethionate.

Examples of non-ionic surfactants that can be used in the present invention are: condensation products of alkylene oxides and alkyl phenols, such as ethoxylated octyl phenol; condensates of ethylene oxide, propylene oxide and ethylenediamine oxide; alkylpolyglucosides; ethers of fatty alcohols and polyols, such as, for example, polyglyceryl-3-hydroxylauryl ether.

Examples of cationic surfactants that can be used in the present invention are: ammonium quaternary salts, in particular ammonium halides, alkyltrimethylammonium halides, dialkyl-dimethylammonium halides, trialkyl-methylammonium halides, carboxylated derivatives of pyrrolidone, such as PCA ethyl-cocoyl-arginate and the salt ethyl-N-cocoyl-L-arginate of D,L-2-pyrrolidone-5-carboxylic acid.

Examples of amphoteric surfactants that can be used in the present invention are: betaines and derivatives, sulfobetaines and derivatives, imidazole derivatives, such as sodium cocoamphodiacetate.

The cosmetic composition according to the present invention also comprises at least one composite powder composed of particles of a crosslinked organosiloxane polymer supporting silica particles on the surface.

The composite powder has a high flowability and a low tendency to aggregate. The particles of crosslinked organosiloxane polymer preferably have an average diameter within the range of 0.5 - 100 micrometres, more preferably 1-20 micrometres.

For the purposes of the present description, the average diameter of the particles of organosiloxane polymer and silica refers to the average number value that can be determined for example by means of Dynamic Light Scattering Analysis (DLSA).

The crosslinked organosiloxane polymer is preferably a crosslinked siliconic elastomer.

The particles of the crosslinked organosiloxane polymer can be obtained in various forms, preferably in spherical form.

Processes for the preparation of crosslinked organosiloxane polymers that can be used for the purposes of the present invention are described for example in EP 0647672 A1 or in US 2010/0209376 A1.

The preparation of the crosslinked organosiloxane polymer can envisage, for example, the reaction of a first organopolysiloxane having at least two alkenyl groups per molecule (compound (I)) with a second organopolysiloxane having at least two hydrogen atoms bound to a silicon atom per molecule (compound (II)).

Compound (I) having at least two alkenyl groups per molecule preferably has the following formula (I) 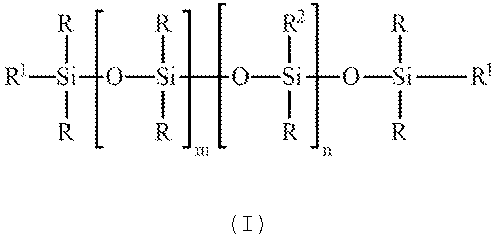
wherein R is a C₁-C₆ alkyl group or a C₆-C₁₀ aryl group,
R¹ is R or a C₂-C₁₀ alkenyl group,
R² is R or a C₂-C₁₀ alkenyl group,
m and n are independently an integer from 0 to 5, 000,
with the proviso that if R¹ is not an alkenyl group, R² is an alkenyl group and n is at least equal to 2.

Compound (II) having at least two hydrogen atoms bound to a silicon atom per molecule preferably has the following formula (II): 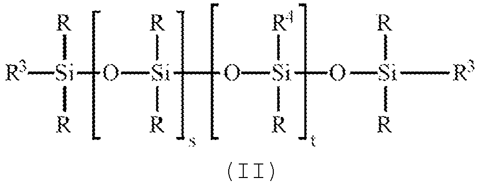
wherein R is a C₁-C₆ alkyl group or a C₆-C₁₀ aryl group,
R³ is R or hydrogen,
R⁴ is R or hydrogen,
s and t are independently an integer from 0 to 5,000,
with the proviso that if R³ is not a hydrogen atom, R⁴ is hydrogen and t is at least equal to 2.

Examples of preferred C₁-C₆ alkyl groups are: methyl, ethyl, propyl, and isopropyl, preferably methyl.

Examples of preferred C₆-C₁₀ aryl groups are: phenyl, naphthyl, preferably naphthyl.

In formulae (I) and (II), R is preferably naphthyl.

Examples of preferred C₂-C₁₀ alkenyl groups are: vinyl, allyl, propenyl, butenyl, pentenyl, hexenyl and decenyl. The alkenyl group is preferably vinyl.

The indexes m, n, s and t preferably have a value within the range of 0-1,000, more preferably 0-200.

The crosslinked organosiloxane polymer is preferably a dimethicone/vinyl dimethicone copolymer.

The particles of the crosslinked organosiloxane polymer support silica particles (SiO₂), preferably amorphous silica. The average diameter (number average value) of the silica particles is preferably within the range of 0.001 - 0.1 micrometres (measured by means of DLSA) .

In order to fix the silica onto the particles of crosslinked organosiloxane polymer, for example, the crosslinked polymer prepared as described above can be dispersed in water to form an aqueous dispersion. Silica is then added to the aqueous dispersion in variable quantities, for example from 0.1 to 30 parts by weight of silica per 100 parts by weight of polymer. The dispersion is then heated, preferably to a temperature within the range of 40-95°C, in order to fix the silica onto the polymer. The water of the dispersion is subsequently removed so as to form a substantially anhydrous composite powder.

A composite powder that can be advantageously used for the purposes of the invention and which is available on the market is the product Dow Corning 9701 Cosmetic Powder (Dow Corning) called INCI dimethicone/vinyl dimethicone.

The overall quantity of composite powder present in the composition is preferably within the range of 0.05-5%, more preferably within the range of 0.1-1%, even more preferably within the range of 0.15-0.5% by weight referring to the weight of the base composition.

The composition according to the present invention can also comprise at least one filler in powder form in a quantity within the range of 1-25% by weight, preferably within the range of 1-10% by weight, with respect to the weight of the base composition. The filler, for example, can be composed of powders of talc, mica, metal oxides (e.g. ZnO) and mixtures thereof.

The particles of filler preferably have an average diameter within the range of 2-40 micrometres (measured by means of DLSA).

The composition can also comprise one or more further ingredients (additives) conventionally used in compositions for the cleansing and/or caring of skin or hair. The additives are preferably incorporated in the composition in the solid state in the form of particles. Before being mixed with the other ingredients of the composition, the liquid additives can be absorbed on a solid ingredient, for example, a filler.

Examples of additives that can be used are: fragrances, preservatives, pigments, dyes, antioxidants, rheology modifiers, pH regulators, sequestering agents, conditioning agents, colorants, cosmetic, dermatological or haircare active ingredients, and mixture thereof.

In a preferred embodiment, the composition comprises panthenol for hair cleansing or care.

The additives are preferably present in the composition in an overall quantity within the range of 0.01-10% by weight, more preferably within the range of 0.1-5% by weight, with respect to the weight of the base composition.

The cosmetic composition according to the present invention can be prepared according to the techniques and with the equipment known to skilled persons in the field.

The powder composition can be obtained by mixing the powders of the various components together, for example in a mixer or mill.

In a preferred embodiment, the process envisages the preliminary preparation of the effervescent system by mixing the base component and the acid component, both in the form of anhydrous powders. The effervescent system is subsequently mixed with the remaining components of the composition. The mixing can be effected in the same mixing apparatus in which the effervescent system was prepared. The mixing of all the components can last, for example, from 1 to 30 minutes.

The powder composition thus formed can be packaged as such in monodose portions (e.g. sachets, capsules, etc.) or multidose portions (e.g. bottles).

If the composition is to be obtained in a self-supporting compact powder form, the powder composition is subjected to compacting after the mixing phase of the components.

The compacting can be effected, for example, with a conventional tablet press. The compacting is preferably carried out at a pressure within the range of 5-200 bar, more preferably 30-100 bar.

The compacting is preferably carried out at room temperature.

In order to apply the composition to the skin, the composition is hydrated. The hydration is preferably effected by adding a weight quantity of water to the composition equal to about 5 to 10 times the weight of the composition. Once the water has been added, the composition is kneaded (for example, in the hollow of a hand) until a creamy fluid is obtained, which can be easily spread with the hands in the form of a thin layer on the skin or applied to the hair. Once the composition has been applied, it can be easily removed by rinsing with water.

The following embodiment example is provided for purely illustrative purposes of the present invention and should not be considered as limiting the protection scope defined by the enclosed claims.

### EXAMPLE

A cosmetic composition was prepared for the cleansing and care of the skin starting from a base composition having the following composition in weight percentage:

| | |
|---|---|
| (a1) sodium bicarbonate (Effersoda) | 58.0% |
| (a2) anhydrous citric acid E330 | 29.0% |
| (b1) sodium lauryl sulfosuccinate (anionic surfactant, Plantapon SUS® BASF) | 5.2% |
| (b2) sodium cocoyl isethionate (anionic surfactant, Jordapon CL Powder, BASF) | 7.5% |
| (c) composite powder (dimethicone/vinyl dimethicone/silica copolymer, Dow Corning 9701). | 0.3% |

The cosmetic composition also contains a fragrance in powder form (2.0% by weight with respect to the weight of the base composition) and zinc oxide as filler (2.0% by weight with respect to the weight of the base composition).

The effervescent system in powder form (sodium bicarbonate and citric acid) was mixed in a mixing mill at about 1500 rpm for 2 minutes. At the end of the mixing, the remaining ingredients of the composition were introduced into the mixing mill. The mixing was re-started and continued for 4 minutes.

The composition was then discharged from the mixing mill and compacted in the form of discoid tablets (1.5 g each) using a hydraulic tablet press and applying a pressure of 40 bar.

The tablets thus obtained can be used, after hydration, for cleansing and/or cosmetically caring the skin. The quantity of composition is suitable for a single treatment for the face or hands.

## Claims

1. Powder cosmetic composition comprising the following components (percentages by weight referred to the total weight of the components (a), (b) and (c):
(a) 60-95% by weight of an effervescent system comprising at least one carbonate or bicarbonate salt of an alkaline metal or alkaline earth metal capable of reacting with at least one acid component, when exposed to water, developing a gas;
(b) 1-35% by weight of at least one solid surfactant;
(c) 0.05-5% by weight of at least one composite powder consisting of particles of a crosslinked organosiloxane polymer supporting silica particles on the surface.

2. Composition according to the preceding claim, wherein said crosslinked organosiloxane polymer is a crosslinked silicone elastomer.

3. Composition according to claim 1, wherein said crosslinked organosiloxane polymer is a dimethicone/vinyl dimethicone copolymer.

4. Composition according to the preceding claim, wherein said composite powder is present in an amount within the range 0.1 - 1% by weight with respect to the total weight of said components (a), (b) and (c), preferably within the range 0.15 - 0.5% by weight.

5. Composition according to any one of the preceding claims, wherein said silica is amorphous silica.

6. Composition according to any one of the preceding claims, wherein said at least one solid surfactant is an anionic surfactant.

7. Composition according to the preceding claim, wherein said at least one solid anionic surfactant is selected from: polyalkylene glycol ethers of fatty alcohols, taurates, acyl lactilates, sodium stearoyl lactylate, alkyl sulfates, sodium lauryl sulfate, polyoxyethylenated alkyl sulphates, alkyl ether sulfate, monoethanolamine lauryl ether sulphate, alkyl ethers carboxylate, monoalkyl phosphate, dialkyl phosphate, arginine mono-(2-esyl decyl) phosphate, ethoxylated alkyl phosphate, N-acyl sarcosinate, sodium lauroyl sarcosinate, sodium myristoyl sarcosinate, N-acylglutamate, sodium lauroyl glutamate, acetyl isethionate, sodium cocoyl isethionate, polysorbate, potassium laurate, potassium myristate, potassium palmitate, potassium stearate and mixture thereof.

8. Composition according to any one of the preceding claims comprising at least one mixture of the following solid anionic surfactants: sodium lauryl sulfosuccinate and sodium cocoyl isethionate.

9. Composition according to any one of the preceding claims comprising at least one powder filler, preferably in an amount within the range 1% - 25% by weight with respect to the total weight of said components (a), (b) and (c).

10. Composition according to any one of the preceding claims further comprising at least one ingredient selected from: fragrances, preservatives, pigments, dyes, antioxidants, rheology modifiers, pH regulators, sequestering agents, conditioning agents, colorants, cosmetic active principles, dermatological active principles or trichological active principles, and mixture thereof.

11. Composition according to any one of the preceding claims in the form of self-supporting compact powder, preferably in the form of a tablet or a bar.

12. Method for cleansing and/or cosmetically caring the skin and/or hair which comprises the steps of:
- hydrating a cosmetic composition according to claim 1 so as to form a foam;
- applying said foam to the skin and/or hair;
- rinsing the skin and/or hair.

13. Process for preparing a cosmetic composition according to claim 1 which comprises a step (i) of mixing the following components (percentages by weight referred to the total weight of the components (a), (b) and (c)):
(a) 60-95% by weight of at least one powder effervescent system comprising at least one carbonate or bicarbonate salt of an alkaline metal or alkaline earth metal capable of reacting with at least one acid component, when exposed to water, developing a gas,
(b) 1-35% by weight of at least one solid surfactant,
(c) 0.05-5% by weight of at least one composite powder consisting of particles of a crosslinked organosiloxane polymer supporting silica particles on the surface,
in order to form said powder cosmetic composition.

14. Process according to the preceding claim comprising the step (ii) of compacting said powder cosmetic composition in order to obtain a cosmetic composition in a self-supporting compact form, preferably in the form of a tablet or a bar.

## Patentansprüche

1. Pulverförmige kosmetische Zusammensetzung, umfassend die folgenden Komponenten (Gewichtsprozentsätze bezogen auf das Gesamtgewicht der Komponenten (a), (b) und (c):
(a) 60-95 Gew.-% eines Brausesystems, umfassend wenigstens ein Karbonat- oder Bikarbonatsalz eines Alkalimetalls oder Erdalkalimetalls, das in der Lage ist, unter Wassereinwirkung mit wenigstens einer Säurekomponente zu reagieren und ein Gas zu entwickeln;
(b) 1-35 Gew.-% wenigstens eines festen Tensids;
(c) 0,05-5 Gew.-% wenigstens eines Kompositpulvers, bestehend aus Partikeln eines vernetzten Organosiloxanpolymers, das Siliciumdioxidpartikel auf der Oberfläche trägt.

2. Zusammensetzung nach dem vorstehenden Anspruch, wobei das vernetzte Organosiloxanpolymer ein vernetztes Silikonelastomer ist.

3. Zusammensetzung nach Anspruch 1, wobei das vernetzte Organosiloxanpolymer ein Dimeticon/Vinyl-Dimeticon-Copolymer ist.

4. Zusammensetzung nach dem vorstehenden Anspruch, wobei das Kompositpulver in einer Menge im Bereich von 0,1-1 Gew.-% in Bezug auf das Gesamtgewicht der Komponenten (a), (b) und (c), vorzugsweise im Bereich von 0,15-0,5 Gew.-% vorliegt.

5. Zusammensetzung nach einem der vorstehenden Ansprüche, wobei das Siliciumdioxid amorphes Siliciumdioxid ist.

6. Zusammensetzung nach einem der vorstehenden Ansprüche, wobei das wenigstens eine feste Tensid ein anionisches Tensid ist.

7. Zusammensetzung nach dem vorstehenden Anspruch, wobei das wenigstens eine feste anionische Tensid ausgewählt ist aus: Polyalkylenglycolethern von Fettalkoholen, Tauraten, Acyllactylaten, Natriumstearoyllactylat, Alkylsulfaten, Natriumlaurylsulfat, polyoxyethylenierten Alkylsulfaten, Alkylethersulfat, Monoethanolamin-Laurylethersulfat, Alkylether-Carboxylat, Monoalkylphosphat, Dialkylphosphat, Arginin-mono-(2-esyl decyl)-Phosphat, ethoxyliertem Alkylphosphat, N-Acylsarcosinat, Natriumlauroylsarcosinat, Natriummyristoylsarcosinat, N- Acylglutamat, Natriumlauroylglutamat, Acetylisethionat, Natriumcocoylisethionat, Polysorbat, Kaliumlaurat, Kaliummyristat, Kaliumpalmitat, Kaliumstearat und Gemischen davon.

8. Zusammensetzung nach einem der vorstehenden Ansprüche, umfassend wenigstens ein Gemisch aus den folgenden festen anionischen Tensiden: Natriumlaurylsulfosuccinat und Natriumcocoylisethionat.

9. Zusammensetzung nach einem der vorstehenden Ansprüche, umfassend wenigstens einen Pulverfüllstoff, vorzugsweise in einer Menge im Bereich von 1 - 25 Gew.-% in Bezug auf das Gesamtgewicht der Komponenten (a), (b) und (c).

10. Zusammensetzung nach einem der vorstehenden Ansprüche, ferner umfassend wenigstens einen Bestandteil ausgewählt aus: Duftststoffen, Konservierungsstoffen, Pigmenten, Farben, Antioxidantien, Rheologiemodifikatoren, pH-Regulatoren, Sequestriermitteln, Konditionierungsmitteln, Farbstoffen, kosmetischen Wirkstoffen, dermatologischen Wirkstoffen oder trichologischen Wirkstoffen und Gemischen davon.

11. Zusammensetzung nach einem der vorstehenden Ansprüche in Form von selbsttragendem Kompaktpuder, vorzugsweise in Form einer Tablette oder eines Riegels.

12. Verfahren zum Reinigen und/oder kosmetischen Pflegen der Haut und/oder des Haares, umfassend die Schritte:
- Hydratisieren einer kosmetischen Zusammensetzung nach Anspruch 1, um einen Schaum zu bilden;
- Auftragen des Schaums auf die Haut und/oder das Haar;
- Abspülen der Haut und/oder des Haares.

13. Verfahren zur Zubereitung einer kosmetische Zusammensetzung nach Anspruch 1, umfassend einen Schritt (i) des Mischens der folgenden Komponenten (Gewichtsprozentsätze bezogen auf das Gesamtgewicht der Komponenten (a), (b) und (c))):
(a) 60-95 Gew.-% wenigstens eines pulverförmigen Brausesystems, umfassend wenigstens ein Karbonat- oder Bikarbonatsalz eines Alkalimetalls oder Erdalkalimetalls, das in der Lage ist, unter Wassereinwirkung mit wenigstens einer Säurekomponente zu reagieren und ein Gas zu entwickeln,
(b) 1-35 Gew.-% wenigstens eines festen Tensids,
(c) 0,05-5 Gew.-% wenigstens eines Kompositpulvers, bestehend aus Partikeln eines vernetzten Organosiloxanpolymers, das Siliciumdioxidpartikel auf der Oberfläche trägt,
um die pulverförmige kosmetische Zusammensetzung zu bilden.

14. Verfahren nach dem vorstehenden Anspruch, umfassend den Schritt (ii) des Kompaktierens der pulverförmigen kosmetischen Zusammensetzung, um eine kosmetische Zusammensetzung in einer selbsttragenden kompakten Form, vorzugsweise in Form einer Tablette oder eines Riegels zu erhalten.

## Revendications

1. Composition cosmétique en poudre comprenant les composants suivants (pourcentages en poids rapportés au poids total des composants (a), (b) et (c) :
(a) 60 à 95 % en poids d'un système effervescent comprenant au moins un sel de carbonate ou de bicarbonate d'un métal alcalin ou d'un métal alcalino-terreux capable de réagir avec au moins un composant acide, lorsqu'il est exposé à de l'eau, développant un gaz ;
(b) 1 à 35 % en poids d'au moins un tensioactif solide ;
(c) 0,05 à 5 % en poids d'au moins une poudre composite constituée de particules d'un polymère d'organosiloxane réticulé portant des particules de silice sur la surface.

2. Composition selon la revendication précédente, dans laquelle ledit polymère d'organosiloxane réticulé est un élastomère de silicone réticulé.

3. Composition selon la revendication 1, dans laquelle ledit polymère d'organosiloxane réticulé est un copolymère diméthicone/vinyl diméthicone.

4. Composition selon la revendication précédente, dans laquelle ladite poudre composite est présente en une quantité comprise dans la plage allant de 0,1 à 1 % en poids par rapport au poids total desdits composants (a), (b) et (c), de préférence dans la plage allant de 0,15 à 0,5 % en poids.

5. Composition selon l'une quelconque des revendications précédentes, dans laquelle ladite silice est de la silice amorphe.

6. Composition selon l'une quelconque des revendications précédentes, dans laquelle ledit au moins un tensioactif solide est un tensioactif anionique.

7. Composition selon la revendication précédente, dans laquelle ledit au moins un tensioactif anionique solide est sélectionné parmi : des éthers de polyalkylène glycol d'alcools gras, des taurates, des acyllactilates, du stéaroyl lactylate de sodium, des sulfates alkyliques, du lauryl sulfate de sodium, des sulfates alkyliques polyoxyéthylénés, du sulfate d'éther alkylique, du lauryl éther sulfate de monoéthanolamine, du carboxylate d'éthers alkyliques, du phosphate de monoalkyle, du phosphate de dialkyle, du phosphate d'arginine mono-(2-esyl décyle), du phosphate alkylique éthoxylé, du N-acyl-sarcosinate, du lauroylsarcosinate de sodium, du myristoyl sarcosinate de sodium, du N-acylglutamate, du lauroyl glutamate de sodium, de l'acétyl iséthionate, de l'iséthionate de cocoyle de sodium, du polysorbate, du laurate de potassium, du myristate de potassium, du palmitate de potassium, du stéarate de potassium et un mélange de ceux-ci.

8. Composition selon l'une quelconque des revendications précédentes comprenant au moins un mélange des tensioactifs anioniques solides suivants : du lauryl sulfosuccinate de sodium et de l'iséthionate de cocoyle de sodium.

9. Composition selon l'une quelconque des revendications précédentes comprenant au moins une charge en poudre, de préférence en une quantité comprise entre 1 % et 25 % en poids par rapport au poids total desdits composants (a), (b) et (c).

10. Composition selon l'une quelconque des revendications précédentes comprenant en outre au moins un ingrédient sélectionné parmi : des parfums, des conservateurs, des pigments, des teintures, des antioxydants, des modificateurs de rhéologie, des régulateurs de pH, des agents séquestrants, des agents de conditionnement, des colorants, des principes actifs cosmétiques, des principes actifs dermatologiques ou des principes actifs trichologiques, et un mélange de ceux-ci.

11. Composition selon l'une quelconque des revendications précédentes sous la forme d'une poudre compacte autoportante, de préférence sous la forme d'un comprimé ou d'une barre.

12. Méthode de nettoyage et/ou de soin cosmétique de la peau et/ou des cheveux qui comprend les étapes consistant à :
- hydrater une composition cosmétique selon la revendication 1 de façon à former une mousse ;
- appliquer ladite mousse sur la peau et/ou les cheveux ;
- rincer la peau et/ou les cheveux.

13. Procédé de préparation d'une composition cosmétique selon la revendication 1 qui comprend une étape (i) consistant à mélanger les composants suivants (pourcentages en poids rapportés au poids total des composants (a), (b) et (c)) :
(a) 60 à 95 % en poids d'au moins un système effervescent en poudre comprenant au moins un sel de carbonate ou de bicarbonate d'un métal alcalin ou d'un métal alcalino-terreux capable de réagir avec au moins un composant acide, lorsqu'il est exposé à de l'eau, développant un gaz,
(b) 1 à 35 % en poids d'au moins un tensioactif solide,
(c) 0,05 à 5 % en poids d'au moins une poudre composite constituée de particules d'un polymère d'organosiloxane réticulé portant des particules de silice sur la surface,
afin de former ladite composition cosmétique en poudre.

14. Procédé selon la revendication précédente comprenant l'étape (ii) consistant à compacter ladite composition cosmétique en poudre afin d'obtenir une composition cosmétique sous une forme compacte autoportante, de préférence sous la forme d'un comprimé ou d'une barre.
